Europäisches Patentamt

(19) European Patent Office

Office européen des brevets

(11) Publication number: **0 040 763**
**B2**

(12) # NEW EUROPEAN PATENT SPECIFICATION

(45) Date of publication of the new patent specification: **10.09.86**

(21) Application number: **81103705.0**

(22) Date of filing: **14.05.81**

(51) Int. Cl.⁴: **C 07 C 51/41,** C 07 C 59/265

(54) Method for the preparation of liquid aluminum citrate.

(30) Priority: **27.05.80 US 153766**

(43) Date of publication of application:
**02.12.81 Bulletin 81/48**

(45) Publication of the grant of the patent:
**07.09.83 Bulletin 83/36**

(45) Mention of the opposition decision:
**10.09.86 Bulletin 86/37**

(84) Designated Contracting States:
**BE DE FR GB NL SE**

(56) References cited:
**DE-C-1 270 540**
**GB-A-1 436 732**
**US-A-3 762 476**

**Zeitschrift Naturforschung Teil b: Anorg.**
**Chem., Org. Chem. Bd. 30b (1975), 446-453**

(73) Proprietor: **MILES LABORATORIES, INC.**
**1127 Myrtle Street**
**Elkhart Indiana 46514 (US)**

(72) Inventor: **Staal, Philip Ward**
**54669 Andover Place**
**Elkhart Indiana 46514 (US)**

(74) Representative: **Jesse, Ralf-Rüdiger, Dr. et al**
**Bayer AG Konzernverwaltung RP**
**Patentabteilung**
**D-5090 Leverkusen 1 Bayerwerk (DE)**

## Description

The removal of oil from underground formations can be divided into three steps. The first step called primary recovery is to allow the oil to flow out of the ground, relying in natural forces or using a simple pumping technique to lift the oil to the surface. The life span of primary recovery operations for a well can vary from only a few years to 30 years or more, depending on the type of formation involved, the viscosity of the crude, the gas content of the oil and the permeability of the reservoir. Under normal conditions, 5 to 25% of the oil in the formation is removed using primary recovery leaving a significant portion of the oil to be removed by more difficult and expensive techniques.

In some fields, the oil saturated formation is contiguous with a larger porous, water containing formation called an aquifier. If this adjoining aquifier outcrops, it will have a continuous supply of salt brine which will feed into the oil reservoir, providing a constant drive to replace the oil removed. The result is as much as 30% of the oil being removed from the reservoir with the economic limit being reached when the oil-water mixture is so high in water that the oil produced does not pay for the pumping and separation costs.

Unfortunately, not all formations are of the above type and secondary recovery operations such as "water flooding" are necessary. In this operation, injection wells are drilled at intervals throughout the field and water is pumped into the oil-bearing formation to displace the oil towards the producing wells. This method has become very popular and results in average total recovery, using both primary and secondary techniques, of 30 to 33%.

The remaining oil, amounting two thirds of the original volume, is the target of tertiary recovery techniques called "Enhanced Oil Recovery" or EOR. One of the first EOR techniques is steam flooding of reservoirs which contain highly viscous crude which is difficult or impossible to remove without raising its temperature to reduce its viscosity. This can be accomplished by injection of high pressure steam or causing underground combustion which results in crude thin enough to be pumped to the surface. Another less popular technique is to pump fluids that mix easily with the oil into the reservoir to dissolve or carry the oil to the producing well. Light hydrocarbons have been used in this technique although $CO_2$ has gained wider favor. In practice, problems with efficiency and uniformity of the $CO_2$ sweep throughout the reservoir have retarded the widespread application of this technique.

A third technique for EOR that is presently gaining acceptance in the field is the use of chemicals and chemical processes to increase oil production. When the reservoir undergoes a waterflood, much of the oil remains behind attached to the walls of the capillary passages and trapped in the pores of the sandstone formation. The waterflood, always following the path of least resistance, bypasses or slides past the oil. A reduction in surface tension by the injection of chemicals can help break this oil loose from the formation. Adding only a surfactant or micellar dispersion, however, does not release much additional oil. After much investigation, several systems were developed to push the surfactant and oil through the formation in a uniform manner. Polymers such as polyacrylamide, carboxymethylcellulose and polysaccharides have been found to possess the desirable properties needed to act as a fluid piston and drive the oil towards the producer well. In practice, the micellar dispersion containing surfactant is pumped into the formation followed by the polymer, which is injected as a fairly linear, low-viscosity molecule in order to permeate the microscopic pores of the sandstone. The polymer is then thickened in the formation by introducing metal ions such as aluminum complexed as aluminum citrate to cross-link the polymer and form a gel. At this point, the gel is moved using water flooding techniques resulting in a sweep of the oil bank towards the producer well. This process is more fully described in United States Patent No. 3,762,476 issued October 2, 1973.

A second system, similar to the micellar-polymer flood but different in its action, involves the injection of a cationic or anionic polymer, depending on the electrical charge of the sandstone in the formation, followed by aluminum citrate to cross-link and gel the polymer. The polymer, by virtue of its ionic charge, is attached to the capillary walls, thereby filling the voids and larger bypass pores while avoiding the smallest passages. The result is a more uniform pore size throughout the formation which results in increased oil production during water flooding.

Aluminum citrate is preferred for use as the cross-linking material because of its low cost and the slow release of aluminum which results in a relatively low period of activity after its injection into the formation. In a typical operation aluminum sulfate hydrate (alum) and sodium citrate dihydrate are dry blended, shipped to the well field, dispersed in water and pumped into the formation. Water dispersion of the dry blend at the well site is necessary due to the unavailability of a suitable method for the preparation of a stable, water solution of aluminum citrate. This method suffers from several disadvantages. First of all, the solid ingredients dissolve slowly and often incompletely in water and the incompletely dissolved material can cause mechanical wear on seals and moving parts of injection pumps. Furthermore, there is no control over the pH of the solution, which is corrosive at its normal pH of about 2.5. In addition, the use of alum introduces sulfate into the formation which when acted upon by sulfate reducing bacteria results in the presence of corrosive quantities of $H_2S$.

In view of the disadvantages of on-site mixing of the dry blend with water prior to injection it would be desirable and it is an object of the present invention to provide a method for the preparation of a stable aqueous solution of aluminum citrate.

The present invention is a method for the preparation of an aqueous solution of an aluminum citrate complex which comprises the steps of:

a) providing an aqueous solution of $AlCl_3$ containing about 34% $AlCl_3$ on a weight/weight basis;

b) combining the $AlCl_3$ solution with an aqueous solution of citric acid, containing about 50% citric acid on a weight/weight basis in sufficient quantity to provide a ratio of aluminum ion to citric acid molecule of about 2:1 while vigorously agitating the resultant solution to form a solution of aluminum citrate containing about 2.9% w/w aluminum; and

c) adding a basic hydroxide of the formula MOH where M is an alkali metal or ammonium cation in aqueous solution to the solution of aluminum citrate to raise the pH of the solution to a level of between about 5.0 and 7.0 while continuing to vigorously agitate the solution and maintaining its temperature at a range of from 20° to 90°C.

The present invention provides the following advantages over the prior art method of dissolving a dry blend of aluminum sulfate and sodium citrate in water at the well site:

1. There is no solids in the solution to cause wear and tear on pumping equipment.

2. The aluminum citrate liquid can be shipped from the formulation site to the well site in tank trucks, stored in bulk and pumped into the formation when desired.

3. There is afforded complete control over the pH of the solution resulting in low corrosion rates and optimum gelling performance.

4. There is no manual handling of dry material required which results in safer operation.

5. There is no opportunity for operator error since the product is complete as it arrives at the well site.

In each of the process steps, there have been discovered certain critical parameters which are necessary to the successful formation of a stable solution.

In a typical formulation, sufficient aluminum chloride is used to provide a mole ratio of aluminum ion to citric acid molecule of 2:1 since citric acid can complex 2 aluminum ions. However, for maximum stability of the solution a ratio of 1.9:1 has been found to be preferable.

Aluminum chloride solutions containing up to 34 weight percent salt are commercially available. Since more concentrated solutions are not stable, this is the maximum practical concentration. Less concentrated solutions are suitable, but since it is desirable to provide an aluminum citrate solution which is highly concentrated yet stable, a concentrated (32° Be) solution is preferred. Likewise, it is desirable to use a citric acid solution which contains up to 50 weight percent citric acid although less concentrated solution can be employed. When mixing the aluminum chloride and citric acid solutions it is essential that vigorous agitation be maintained throughout the process until a clear solution of aluminum citrate is achieved. At this point, the solution's pH is too low for compatibility with its use in the above described enhanced oil recovery techniques and the addition of an alkali metal or ammonium hydroxide is called for. This is accomplished by adding an aqueous solution of the basic material to the aluminum citrate solution in sufficient quantity to raise its pH to a level of from 5.0 to 7.0, preferably from 6.0 to 7.0. Alternatively, ammonia gas can be bubbled into the solution to form ammonium hydroxide *in situ*. When dry ammonia is used as the source of the base, some dilution of the aluminum citrate solution is desirable to avoid precipitation. Vigorous agitation of the solution is essential during the addition of the base to prevent precipitation of the aluminum already in solution in the form of aluminum hydroxide which will reduce the aluminum assay of the solution. In addition, temperature control is essential during this step due to the exothermic reaction which results upon addition of the base. The temperature should be maintained within the range of from 20° to 90°C during this step with a temperature in the range of from 40° to 60°C being preferred. Slow addition of the base together with external cooling have been found adequate to maintain the temperature within the desired.

An aluminum citrate solution prepared in the above described manner will remain stable for a period of from 10 to 14 days. It has been discovered that the period of stability can be increased by adding sufficient base to raise the pH to a level of 6.0 to 7.0 and then lowering the pH to a level of from 5.0 to 5.5 by the addition of a mineral acid, preferably HCl.

The method of practicing the present invention is further illustrated by the following examples:

### Example I

A 600 ml beaker was charged with 258 g of a commercially available 34 weight % $AlCl_3$ solution and 107.65 g of a 50% citric acid solution to provide a mole ratio of aluminum to citric acid of 2:1. The resulting solution was stirred vigorously for 35 minutes after which period 25 ml of a 50% NaOH solution was added via burette over a period of 36 minutes.

This procedure resulted in a liquid aluminum citrate having the following specifications:

| | |
|---|---|
| Aluminum Assay | = 2.9% w/w (3.7% w/v) |
| pH | = 6.8 |
| Sq. Gravity | = 1.295 |
| Freezing (crystallization Point) | = −20°C |

This solution remained stable for a period of approximately 2 weeks.

### Example II

The following experiment was run to develop a formulation based on 2.0 moles of Al to 1.1 mole of citric acid:

A 250 ml beaker was charged with 55 g of a 34% solution of aluminum chloride. To this was quickly added 25.3 g of a 50% (w/w) citric acid solution and 30.0 g of a 50% NaOH solution was added, while keeping the temperature at about 60°C, to bring the pH to 6.5. This procedure provided a crystal clear solution having a specific gravity of 1.293 which remained stable for approximately 2 weeks.

### Example III

The following procedure was carried out in a 21,196 liter, fiberglass fix tank equipped with 10 coils of a 3.8 cm diameter polyvinyl chloride cooling coil, a turbine agitator and one air wand for additional agitation.

The procedure was as follows:

### Day 1

4:30 PM The tank was charged with 7,986 liters of a 34% aluminum chloride solution directly from a tank truck.

7:30 PM Citric acid, 3,857 liters of a 50% (w/w) solution, was added while maintaining vigorous agitation.

9:00 PM Slow addition of a 50% (w/w) NaOH solution was commenced with external cooling of the vat. At this point the pH was less than 1 and the temperature was 55°C. Addition of the sodium hydroxide was continued for approximately 18 hours under the following schedule:

| Day 2 | Temperature | pH |
|---|---|---|
| 7:30 AM | 55°C | — |
| 4:00 PM | 53°C | 1.24 |
| 8:30 PM | 54°C | 2.76 |
| 12:00 Midnight | 54°C | 4.72 |
| Day 3 | Temperature | pH |
| 12:18 AM | 54°C | 5.39 |
| 12:44 AM | 55°C | 6.05 |
| 1:01 AM | 55°C | 6.20 |
| 1:15 AM | 55°C | 6.40 |
| 1:30 AM | — | 6.57 |

By the time the desired pH was achieved the total amount of NaOH added was 3,709 liters. This procedure provided 15,140 liters of liquid ammonium citrate containing 2.97% (w/w) aluminum.

After two weeks, a sample of the material became cloudy. Analysis of the supernatant showed 2.74% (w/w) aluminum indicating that 7.7% of the original aluminum had precipitated out.

## Claims

1. A method for the production of a concentrated liquid aluminum citrate which comprises the steps of:

a) providing an aqueous solution of $AlCl_3$ containing about 34% $AlCl_3$ on a weight/weight basis;

b) combining the $AlCl_3$ solution with an aqueous solution of citric acid, containing about 50% citric acid on a weight/weight basis in sufficient quantity to provide a ratio of aluminum ion to citric acid molecule of about 2:1 while vigorously agitating the resultant solution to form a solution of aluminum citrate containing about 2.9% w/w aluminum; and

c) adding a basic hydroxide of the formula MOH where M is an alkali metal or ammonium cation in aqueous solution to the solution of aluminum citrate to raise the pH of the solution to a level of between about 5.0 and 7.0 while continuing to vigorously agitate the solution and maintaining its temperature at a range of from 20° to 90°C.

2. The method of claim 1 wherein the ratio is 1,9:1.

3. The method of claims 1—2 wherein the temperature is maintained at a level of from 40°C to 60°C during the addition of the basic hydroxide.

4. The method of claim 1 wherein the basic hydroxide is NaOH.

5. The method of claim 4 wherein the NaOH is added in the form of its 50% (w/w) aqueous solution.

6. The method of claims 1—5 which comprises the steps of:

a) providing an aqueous solution of aluminum chloride containing about 34% (w/w) of aluminum chloride;

b) combining the aluminum chloride solution with an aqueous solution of citric acid containing about 50% (w/w) citric acid while providing vigorous agitation to form a solution of aluminum citrate;

c) adding a 50% (w/w) solution of sodium hydroxide to the aluminum citrate solution, while continuing to provide vigorous agitation and maintaining the temperature of the resultant at a level of from 40°C to 60°C, to raise the pH to level of from 6.0 to 7.0.

## Patentansprüche

1. Verfahren zur Herstellung von konzentriertem flüssigen Aluminiumcitrat, gekennzeichnet durch folgende Stufen:

(a) Herstellen einer wässrigen Lösung von $AlCl_3$ mit einem Gehalt von etwa 34% $AlCl_3$ auf einer Gewicht/Gewickt-Basis;

(b) Kombinieren der $AlCl_3$-Lösung mit einer wässrigen Lösung von Citronensäure, enthaltend etwa 50% Citronensäure auf einer Gewicht/Gewicht-Basis in einer ausreichenden Menge, um das Verhältnis von Aluminiumion zu Citronensäuremolekül auf etwa 2:1 einzustellen, wobei man die erhaltene Lösung kräftig rührt unter Ausbildung einer Lösung von Aluminiumcitrat, enthaltend etwa 2,9% Gewicht/Gewicht Aluminium; und

(c) Zugabe eines basischen Hydroxids der Formel MOH, worin M ein Alkalimetall oder ein Ammoniumkation bedeutet, in einer wässrigen Lösung zu der Lösung des Alluminiumcitrats unter Erhöhung des pH-Wertes der Lösung auf ein Niveau zwischen etwa 5,0 und 7,0, wobei man weiterhin kräftig die Lösung rührt und deren Temperatur in einem Bereich von 20 bis 90°C hält.

2. Verfahren gemäss Anspruch 1, worin das Verhältnis 1,9:1 beträgt.

3. Verfahren gemäss Ansprüchen 1 bis 2, worin die Temperatur auf einem Niveau von 40 bis 60°C während der Zugabe des basischen Hydroxids gehalten wird.

4. Verfahren gemäss Anspruch 1, worin das basische Hydroxid NaOH ist.

5. Verfahren gemäss Anspruch 4, worin die zugegebene NaOH in Frm einer 50%-igen (G/G) wässrigen Lösung vorliegt.

6. Verfahren gemäss Ansprüchen 1 bis 5, gekennzeichnet durch folgende Stufen:

(a) Herstellen einer wässrigen Lösung von Aluminium chlorid, enthaltend etwa 34% (G/G) Aluminiumchlorid;

(b) Kombinieren der Aluminiumchloridlösung mit einer wässrigen Lösung von Citronensäure, enthaltend etwa 50% (G/G) Citronensäure, während man kräftig rührt, unter Ausbildung einer Lösung von Aluminiumcitrat;

(c) Zugabe einer 50%-igen (C/G) Lösung von Natriumhydroxid zu der Aluminiumcitratlösung, während man kräftig rührt und die Temperatur der erhaltenen Mischung auf einem Niveau von 40 bis 60°C hält, unter Erhöhung des pH-Wertes auf ein Niveau von 6,0 bis 7,0.

**Revendications**

1. Procédé pour la préparation de citrate d'aluminium liquide qui comprend les étapes suivantes:

a) on part d'une solution aqueuse de AlCl$_3$ contenant environ 34% en poids de AlCl$_3$;

b) on combine la solution de AlCl$_3$ avec une solution aqueuse d'acide citrique contenant environ 50% en poids d'acide citrique en quantité suffisante pour donner un rapport ions aluminium/molécules d'acide citrique d'environ 2:1, tout en agitant vigoureusement la solution résultante, pour former une solution de citrate d'aluminium contenant environ 2,9% en poids d'aluminium; et

c) on ajoute à la solution de citrate d'aluminium un hydroxide basique de formule MOH, où M est un cation de métal alcalin ou d'ammonium pour élever le pH de la solution à une valeur d'environ 5,0 à 7,0, tout en continuant à agiter vigoureusement la solution et en maintenant sa température dans la gamme de 20 à 90°C.

2. Procédé selon la revendication 1, dans lequel le rapport est de 1,9:1.

3. Procédé selon les revendications 1—2, dans lequel le température est maintenue à une valeur de 40 à 60°C pendant l'addition de l'hydroxyde basique.

4. Procédé selon les revendications 1, dans lequel l'hydroxyde basique est NaOH.

5. Procédé selon la revendication 1, dans lequel NaOH est ajouté sous forme de sa solution aqueuse à 50% (en poids).

6. Procédé selon les revendications 1—5, qui comprend les étapes suivantes:

a) on part d'une solution aqueuse de AlCl$_3$ contenant environ 34% (en poids) de AlCl$_3$;

b) on combine la solution de AlCl$_3$ avec une solution aqueuse d'acide citrique contenant environ 50% (en poids) d'acide citrique tout en agitant vigoureusement pour former une solution de citrate d'aluminium; et

c) on ajoute à la solution de citrate d'aluminium une solution à 50% (en poids) d'hydroxyde de sodium, tout en continuant à agiter vigoureusement et en maintenant la température de la solution résultante dans la gamme de 40 à 60°C poure élever le pH de la solution à une valeur de 6,0 à 7,0.